# EUROPEAN PATENT SPECIFICATION

(11) **EP 1 769 240 B1**
(45) Date of publication and mention of the grant of the patent: **09.11.2011**
(21) Application number: 05757530.0
(22) Date of filing: 17.06.2005
(51) Int. Cl.: C12Q 1/37, C12N 9/50, C12N 9/52, C12N 9/54

(54) **PURIFICATION METHODS AND USES THEREOF**
REINIGUNGSVERFAHREN UND IHRE VERWENDUNGEN
PROCEDES DE PURIFICATION ET LEURS UTILISATIONS

(30) Priority: 17.06.2004 NZ 53363404
(43) Date of publication of application: 04.04.2007
(73) Proprietor: ZyGEM Corporation Limited, Hamilton (NZ)
(72) Inventor: DANIEL, Roy McIver, Hamilton (NZ); SAUL, David James, Palm Beach, Auckland (NZ)
(74) Representative: Brasnett, Adrian Hugh
(86) International application number: PCT/NZ2005/000133
(87) International publication number: WO 2005/122709

(56) References cited:
- WO-A-03/064605
- WO-A1-02/092844
- US-A1- 2002 177 139
- US-B1- 6 469 159
- SAUL D J ET AL: "SEQUENCE OF THE GENE ENCODING A HIGHLY THERMOSTABLE NEUTRAL PROTEINASE FROM BACILLUS SP. STRAIN EA1: EXPRESSION IN ESCHERICHIA COLI AND CARACTERISATION" BIOCHIMICA ET BIOPHYSICA ACTA, AMSTERDAM, NL, vol. 1308, no. 1, 31 July 1998 (1998-07-31), pages 74-80, XP001191007 ISSN: 0006-3002
- TOOGOOD H S ET AL: "PURIFICATION AND CHARACTERIZATION OF AK.1 PROTEASE, A THERMOSTABLE SUBTILISIN WITH A DISULPHIDE BOND IN THE SUBSTRATE-BINDING CLEFT" BIOCHEMICAL JOURNAL, THE BIOCHEMICAL SOCIETY, LONDON, GB, vol. 350, no. PART 1, 2000, pages 321-328, XP001191006 ISSN: 0264-6021
- MOSS D ET AL: "An easily automated, closed-tube forensic DNA extraction procedure using a thermostable proteinase." INTERNATIONAL JOURNAL OF LEGAL MEDICINE, vol. 117, no. 6, December 2003 (2003-12), pages 340-349, XP002476705 ISSN: 0937-9827

## Description

### TECHNICAL FIELD

This invention relates to improved methods for isolation, purification, diagnostic, analytical and manufacturing techniques. In particular, the methods of the invention will facilitate improved purification, diagnostic, analytical and manufacturing techniques, especially those in the field of molecular biology, by providing means of removing contamination and/or undesired constituents, and/or by simplifying the techniques.

Preferably the contamination that is removed using the methods of the present invention includes the substrates of enzymes, and proteins, including enzymes, such as for example, nucleic acid-modifying enzymes. In preferred embodiments, the purification methods are directed to reducing unwanted nucleic acid and/or nucleic acid-modifying enzymes in solutions and samples. It is envisaged that such purified solutions and samples will greatly enhance procedures involving nucleic acid and the results obtained therefrom. However, the invention has application outside this field.

### BACKGROUND

Contamination is a significant problem in most isolation, purification, diagnostic, analytical and manufacturing methods, and particularly so where such methods are sensitive. Such contamination is undesirable, leading to less robust methodologies and potentially equivocal results. For example, a number of molecular biological techniques are highly sensitive to contamination, and this contamination may lead to a false positive or a false negative result. For example, PCR techniques are capable of yielding a significant multiplication of target molecules, and are thus highly sensitive to the presence of contaminating molecules, even where pure test organisms and purportedly ultra-pure working solutions are used. Indeed, contaminating nucleic acid from contaminating organisms remains even though the organisms may be dead and the solutions sterile. The results of diagnostic or analytic methods reliant on PCR techniques cannot be considered unequivocal where contamination has occurred or is likely to occur.

Contamination also results where components are added to a composition during a procedure, but cannot be readily removed from the composition prior to subsequent use, for example, the next step of the procedure. For example, an agent may be added to a sample composition to perform a desired function, but may be carried over into subsequent steps of a procedure in which that function is no longer desirable. For example, a restriction enzyme may be added to a nucleic acid sample to effect digestion of the nucleic acid, and must be removed from the nucleic acid sample prior to subsequent use of the sample.

Indeed, contamination of compositions, such as samples and/or working solutions, may occur simply as a result of the reagent tubes being opened to the atmosphere or being touched by a technician. In addition, contamination may arise during preparation of the working solutions. Contaminants such as nucleic acids may also remain attached to glassware, containers and the like, even after strenuous cleaning, sterilization, and stringent washing procedures.

The accuracy and reproducibility of many diagnostic, analytical and manufacturing techniques, and particularly current molecular biology techniques including, for example, genotyping and genomic analyses, pathogen detection and monitoring, and forensic identification, are frequently dependent at least in part on the purity, quantity, and quality of the sample being analyzed, and the compositions and materials used in the technique.

Applications of nucleic acid diagnostic techniques range from forensic and evidential analyses to medical, agricultural and environmental monitoring. It is critical that any working solutions are free from contamination, particularly where the concentration of the nucleic acid in the initial sample is very low and where contamination can lead to incorrect outcomes. This is particularly the case in forensic and evidential analysis where quantities of sample template may be measured in picograms or less. Furthermore, many nucleic acid techniques are sensitive to the carry-over of components from one procedural step to the next.

Minimising and preferably negating the risk of contamination of compositions during isolation, purification, diagnostic, analytical and manufacturing procedures is clearly of prime importance. Similarly, maximising the removal of contaminants, including undesired components including those components whose continued presence in or on said composition is or becomes undesireable, present in or on a composition without the prospect of introducing further contamination is of critical importance.

While techniques exist for the removal of contaminants from compositions, these techniques typically require the subsequent addition to the composition of an agent capable of removing the existing contamination. This addition can itself introduce further contamination.

At present, there is no generally applicable way of ensuring a composition is free of contamination, nor of effecting a removal of contamination without the attendant risk of introducing yet further contamination. A method to remove cell walls utilising a combination of a mesophilic enzyme and a thermophilic enzyme is described in WO02/00093. However, the method is reliant on a mesophilic enzyme having activity effective to remove cell walls, and the methods described are for the preparation of nucleic acid samples for testing.

In the context of methods reliant on PCR for example, there is no way of ensuring reagents and working solutions are free of nucleic acid contamination before PCR. Indeed, current methods directed to a reduction of contamination in PCR techniques are generally reliant on the use of ultra-pure water and reagents to prepare solutions, and careful handling of samples and reagents.

It would be therefore be advantageous to have a method of removing contamination from a composition without the risk of introducing further contamination, preferably wherein the method further allows the removal from the composition of undesirable components or activity added to effect the removal of said contamination, again without risk of introducing further contamination.

It would also be advantageous to have a procedure for purifying the materials and solutions used for PCR whereby contaminating nucleic acid and/or nucleic acid-degrading enzymes could be minimised and/or removed, without the risk of introducing further contamination. It would further be advantageous to have a procedure where the procedure for ensuring purification of the working solutions does not entail the introduction of more complex or time consuming steps in the overall PCR process.

It is an object of the present invention to at least address the foregoing problems or at least to provide the public with a useful choice.

### SUMMARY OF THE INVENTION

According to a first aspect, the present invention provides the use of at least one thermophilic protease and one or more mesophilic enzyme according to claim 1.

Disclosed herein is the use of at least one thermophilic protease and one or more mesophilic enzyme that does not exhibit activity effective to remove cell walls in combination in the removal of at least one contaminant from a composition, wherein said contaminant is a substrate of said one or more mesophilic enzyme.

In one embodiment, said thermophilic protease is selected from the group comprising EA1, Ak1, NprS, NprT, BT1, YP-T, aqualysin I, NprM, Caldolysin, Caldolase,Rt41A, RT4A2, and variants thereof, preferably said thermophilic protease is EA1 or Ak1 or a variant thereof.

Disclosed herein is the decontaminated product of the above use.

According to a further aspect, the present invention provides a method for the removal of at least one contaminant from a composition, wherein said contaminant is a substrate of one or more mesophilic enzyme, said method comprising the steps of:
adding to the composition said one or more mesophilic enzyme and at least one thermophilic protease; and
maintaining said composition at a first temperature or temperature range at which said one or more mesophilic enzyme has enzymatic activity, but at which said at least one thermophilic protease has minimal or no proteolytic activity; and
maintaining said composition at a second temperature or temperature range at which said at least one thermophilic protease has proteolytic activity; and
maintaining said composition at a temperature suitable for use;
wherein said one or more mesothermic enzyme does not exhibit activity effective to remove cell walls.

It will be appreciated that maintaining said composition at a temperature or temperature range includes both maintaining said composition at a single, discrete temperature and maintaining said composition generally within the contemplated range of temperatures.

In one embodiment, said method may involve after said maintenance at a second temperature or temperature range one or more repetitions of the additional steps of:
adding to the composition one or more mesophilic enzyme; and
maintaining said composition at a temperature or temperature range at which said one or more mesophilic enzyme has enzymatic activity, but at which said at least one thermophilic protease has minimal or no proteolytic activity; and
maintaining said composition at a temperature or temperature range at which said at least one thermophilic protease has proteolytic activity.

In another embodiment, said method comprises the additional step of maintaining said composition at a third temperature or temperature range sufficient to cause denaturation and/or autolysis of said at least one thermophilic protease.

Preferably, said first temperature or temperature range is between about 10°C to about 65°C, more preferably between about 20°C and about 50°C, more preferably between about 30°C and about 40°C.

Preferably, said second temperature or temperature range is between about 65°C and about 85°C, more preferably between about 65°C and about 80°C, more preferably between about 70°C and about 80°C, more preferably about 75°C.

Preferably, said third temperature or temperature range is above about 90°C, more preferably between about 90°C and about 105°C, more preferably between about 90°C and about 100°C, more preferably between about 90°C and about 95°C, more preferably about 95°C.

Preferably, said one or more mesophilic enzyme is a nucleic acid-modifying enzyme, more preferably said enzyme is selected from the group comprising kinases, polymerases, synthetases, ligases, methylases, transferases and recombinases.

In a preferred embodiment, said composition is or is in a closable container, more preferably said container remains closed after addition of said one or more mesophilic enzyme and said at least one thermophilic protease and prior to use. In a further preferred embodiment, said method is performed in a closed tube system.

In a further preferred embodiment, the method of the invention is automated, preferably said method utilises a thermal cycler, more preferably utilises a thermal cycler in combination with robotic liquid handling, more preferably said method utilises a robotic workstation.

According to another aspect, the present invention provides a method for the removal of at least one contaminant from a composition, wherein said contaminant is a substrate of one or more mesophilic enzyme, said method comprising the steps of:
adding to the composition said one or more mesophilic enzyme and at least one thermophilic protease; and
maintaining said composition at a first temperature or temperature range of from 40°C to about 65°C at which said one or more mesophilic enzyme has enzymatic activity; and
maintaining said composition at a second temperature or temperature range at which said at least one thermophilic protease has proteolytic activity; and
maintaining said composition at a temperature suitable for use.
In one embodiment, said mesophilic enzyme is a nucleic acid-modifying enzyme, more preferably said enzyme is selected from the group comprising nucleases, phosphatases, kinases, polymerases, synthetases, ligases, methylases, transferases and recombinases.

According to yet another aspect, the present invention provides a method for the removal of nucleic acid from a composition, said method comprising the steps of:
adding to the composition one or more mesophilic nucleic acid-degrading enzyme and at least one thermophilic protease; and
maintaining said composition at a first temperature or temperature range at which said one or more mesophilic nucleic acid-degrading enzyme has enzymatic activity, but at which said at least one thermophilic protease has minimal or no proteolytic activity; and
maintaining said composition at a second temperature or temperature range at which said at least one thermophilic protease has proteolytic activity; and
maintaining said composition at a temperature suitable for use.

Preferably, said one or more mesophilic nucleic acid-degrading enzyme is a nuclease.

According to a further aspect of the present invention there is provided a method for purifying a working solution for nucleic acid diagnostic techniques in a closed-tube system,
said method comprising the steps of:
adding to the working solution one mesophilie nuclease and at least one thermophilic protease capable of degrading potentially contaminating nucleic acid and/or nucleic acid degrading enzymes in said working solution; and
storing said working solutions at a preferred temperature until required for use; and
completing purification prior to using the solution by raising the temperature of said working solution; and
cooling the working solution for use,
the method characterised by the use of both a mesophilic nuclease and a thermophilic protease having differing temperature activity profiles.

According to another aspect of the present invention there is provided a method for purifying a working solution for nucleic acid diagnostic techniques substantially as described above wherein said techniques include the polymerase chain reaction techniques.

Disclosed herein is a method for purifying a working solution for nucleic acid diagnostic techniques substantially as described above, wherein the thermophilic protease is sourced and/or derived from *Bacillus* sp. as herein defined.

Disclosed herein is a method for the rapid preparation of samples for nucleic acid diagnostic techniques, substantially as described above, wherein said *Bacillus* derived proteases include a *Bacillus* sp. str. EA1, being a neutral protease, and *Bacillus* sp. str. Ak1, being a serine protease.

According to still another aspect of the present invention there is provided a method for purifying a working solution for nucleic acid diagnostic techniques substantially as described above, wherein the working solutions may undergo more than one heat treatment step.

Disclosed herein is a method for purifying working solutions for nucleic acid diagnostic techniques substantially as described above, wherein the temperature to which the partially purified working solutions are heated to is ≥ 90°C.

Disclosed herein is the decontaminated composition and/or working solution of any of the methods above.

The invention consists in the foregoing and also envisages constructions of which the following gives examples only.

### BRIEF DESCRIPTION OF DRAWINGS

Further aspects of the present invention will become apparent from the following description which is given by way of example only and with reference to the accompanying drawings in which:

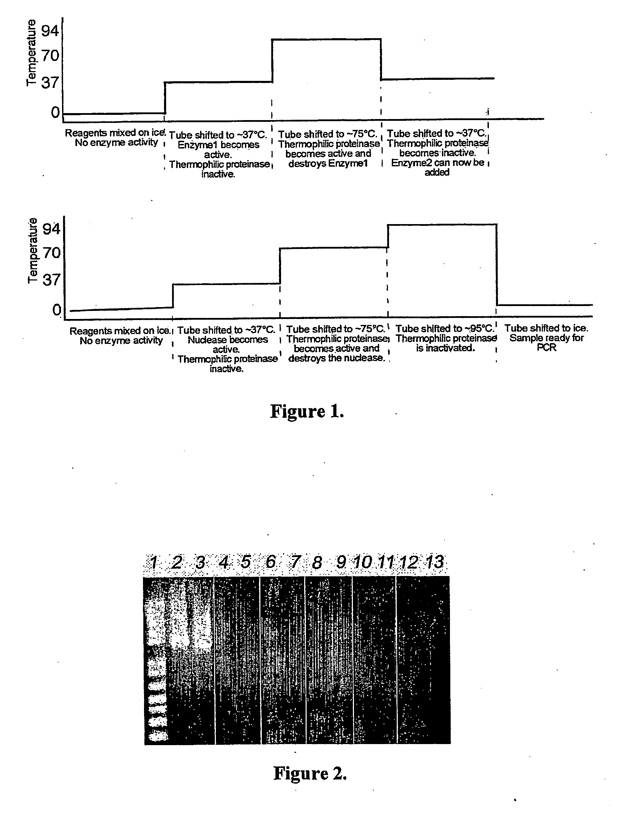

- Figure 1: is a diagrammatical illustration of the different activities that occur at different temperatures in the purification of reagent solutions in accordance with one preferred embodiment of the present invention. The upper panel depicts an exemplary procedure where a further mesophilic enzyme-catalysed step is conducted following the inactivation of the initial one or more mesophilic enzyme by the thermophilic protease. The lower panel depicts an exemplary procedure where a single mesophilic enzyme-catalysed step is conducted whereupon the one or more mesophilic enzyme is degraded by the thermophilic protease, which is itself then degraded prior to sample use.
- Figure 2: is a gel image of various PCRs illustrating the removal of contaminating DNA from solutions. 1 kb+ DNA ladder (lane 1), positive PCR control (lane 2 and 3), 20^{th} cycle (lane 4 and 5), 25^{th} cycle (lane 6 and 7), 30^{th} cycle (lane 8 and 9), 35^{th} cycle (lane 10 and 11), 40^{th} cycle (lane 12 and 13); see Example 1 herein.

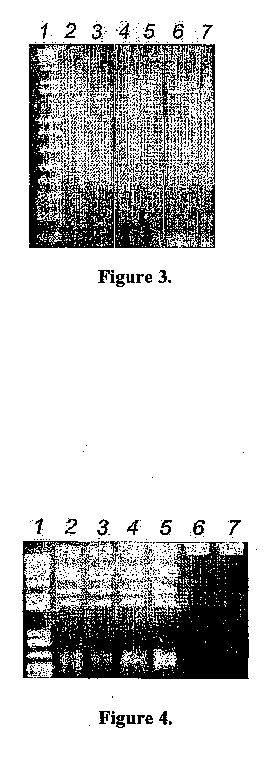

- Figure 3: is a gel image of various PCRs illustrating the removal of mesophilic enzyme from samples. 1 kb+ DNA ladder (lane 1), Positive PCR control (lanes 2 and 3), DNAse 1 only (lane 4 and 5), EA1 thermophilic protease + DNAse1 (lane 6 and 7); see Example 1 herein.
- Figure 4: is a gel image of various enzymatic reactions illustrating the removal of heat-resistant mesophilic enzyme. lkb+ DNA ladder (lane 1), *Pvu*II no treatment incubated with plasmid DNA (lane 2 and 3), *Pvu*II heat treated at 75°C for 15 minutes incubated with plasmid DNA (lane 4 and 5), *Pvu*II heat treated at 75°C in the presence of EA1 thermophilic protease incubated with plasmid DNA (lane 6 and 7); see Example 3 herein.

### DETAILED DESCRIPTION OF THE INVENTION

The methods of the present invention allow the enzymatic catalysis of a substrate of one or more mesophilic enzyme present in or on a composition, followed by the removal from said composition of said one or more mesophilic enzyme by at least one thermophilic protease. The present invention recognizes that the activity of enzyme and protease can be manipulated by varying the temperature of the composition: by maintaining the composition at a temperature at which a mesophilic enzyme is active, but at which a thermophilic protease exhibits minimal or preferably no activity, the substrate of a mesophilic enzyme can be catalysed; then, by increasing the temperature of the composition, the activity of the thermophilic protease can be increased to effect proteolysis of said mesophilic enzyme. Advantageously, the differing temperature activity profiles of the mesophilic enzyme and the thermophilic protease allows the concurrent addition of mesophilic enzyme and thermophilic protease. This minimises handling, allows for ready automation, and reduces the opportunity for and risk of contamination, wherein the methods of the invention are particularly amenable to implementation in closed systems, for example, a closed-tube system.

It is envisaged that in certain embodiments, more than one mesophilic enzyme may be added to a composition, for example to effect the catalysis of more than one reaction such as the removal of more than one substrate, including, for example, where the product of one mesophilic enzyme is the substrate of another. Similarly, in various embodiments more than one thermophilic protease can be added to a composition.

Accordingly, in one aspect, the present invention provides the use of a combination of at least one thermophilic protease and one or more mesophilic enzyme according to claim 1.

As used herein, the phrase "nucleic acid for testing" means nucleic acid that is or is to be the subject of a diagnostic or analytical test.

In a further aspect, the invention provides a method for the removal of at least one contaminant from a composition, wherein said contaminant is a substrate of one or more mesophilic enzyme, said method comprising the steps of:
adding to the composition said one or more mesophilic enzyme and at least one thermophilic protease; and
maintaining said composition at a first temperature or temperature range at which said one or more mesophilic enzyme has enzymatic activity, but at which said at least one thermophilic protease has minimal or no proteolytic activity; and
maintaining said composition at a second temperature or temperature range at which said at least one thermophilic protease has proteolytic activity; and
maintaining said composition at a temperature suitable for use.

It will be appreciated that the uses and methods of the invention readily allow the removal of one or more contaminant from compositions, provided said contaminant is a substrate of a mesophilic enzyme. The invention recognises that such a mesophilic enzyme can itself be considered a contaminant, and enables the removal of such a mesophilic enzyme through the activity of a thermophilic protease. The invention further recognises that any residual proteolytic activity present in or on the composition may be undersirable, such that thermophilic proteases suitable for use in the methods of the invention are heat denaturable and/or exhibit autolytic activity, and are preferably permanently heat-inactivated at elevated temperatures, for example, at temperatures at or above about 90°C.

It will further be appreciated that the methods of the invention allows the (possibly repeated) activation and reversible inactivation of the thermophilic protease. For example, if the procedure requires other, subsequent mesophilic enzyme-mediated reactions following the inactivation of a first mesophilic enzyme by the protease, then reducing the composition to a temperature at which the thermophilic protease exhibits no or minimal proteolytic activity enables the addition of further mesophilic enzyme and its catalytic activity. Removal of mesophilic enzyme can then be effected by maintaining the composition at a temperature at which the thermophilic protease is active. Advantageously, the thermophilic protease can itself be irreversibly deactivated if required. For example, in circumstances where subsequent reactions use thermophilic enzymes (for example *Taq* DNA polymerase) which for activity require incubation at temperatures at which the thermophilic protease exhibits activity, then the protease can be irreversibly denatured at temperatures above about 90°C, such as 95°C.

The characteristics of the enzymes utilised in the present invention, such as for example their activity and stability, can also be manipulated by manipulating the conditions of the composition to, for example, control the temperature at which, for example, activity, denaturation, autolysis and the like occur. For example, many enzymes are more stable in high concentrations of calcium (for example, hundreds of millimolar) than in low or no calcium (for example, sub micromolar). For example, by maintaining the composition under low calcium concentration conditions the enzyme including the thermophilic protease can be destabilised and can be destroyed more quickly and/or at a lower temperature than at high calcium concentrations. Similarily, the inclusion of agents which chelate calcium (such as EDTA) and thus make it unavailable to the enzyme or protease or remove it from the enzyme or protease, will also destabilise the enzyme or protease. It will be appreciated that by manipulating the conditions at which the composition is maintained, the activity of the one or more mesophilic enzyme and/or the at least one thermophilic protease can be controlled, for example, to control the temperature at which the protease will act to degrade the added and contaminating mesophilic enzyme while remaining intact itself.

The term "comprising" as used in this specification means "consisting at least in part of". When interpreting statements in this specification which include that term, the features, prefaced by that term in each statement or claim, all need to be present but other features can also be present.

As used herein, the term "activity effective to remove cell walls" means an enzymatic activity capable of degrading cell walls that is exhibited under the conditions maintained in the methods of the invention.

As used herein, the term "mesophilic enzyme" includes enzymes having optimal enzymatic activity at temperatures below that at which the thermophilic protease has optimal enzymatic activity, for example, below about 65°C, preferably from about 0°C to about 65°C, more preferably from about 10°C to about 65°C, and includes enzymes isolated from mesophilic organisms (generally referred to as mesophilic enzymes), and enzymes isolated from organisms other than mesophilic organisms that have optimal enzymatic activity as described above, including any such enzymes that have subsequently been modified or engineered and those of such stability and other properties as to allow activity in the temperature range of 0°C to about 65°C. For a discussion of the factors affecting the temperature range for enzymatic activity, see Daniel RM, et al., "The temperature optima of enzymes; a new perspective on an old phenomenon." Trends Biochem. Sci., 26: 223-225 (2001), and Peterson ME, et al., "A new, intrinsic, thermal parameter for enzymes reveals true temperature optima." J Biol Chem, 279: 20717-20722 (2004)).

Preferably, said mesophilic enzyme is a nucleic acid-modifying enzyme, more preferabley said mesophilic enzyme is selected from the group comprising nucleases, phosphatases, kinases, polymerases, synthetases, ligases, methylases, transferases and recombinases.

In another preferred embodiment, said mesophilic enzyme is selected from the group comprising ATPases, cytochromes, deaminases, dehydrogenases, decarboxylases, desaturases, dioxygenases, elastases, endopeptidases, flavoproteins, flippases, hydrogenases, hydrolases, hydroxylases, integrases, isomerases, kinases, ligases, lipases, lipoxygenases, lyases, mutases, oxidases, oxioreductases, peptidases and proteolytic enzymes, permeases, peroxidases, phosphorylases, recombinases, reductases, regulatory enzymes, ribonucleases, sulfatases, synthases, synthetases, telomerases, and transferases.

In addition a thermophilic protease is preferably added concurrently with the nuclease in this initial decontamination step. The particular thermophilic protease may be obtained from any preferred thermophilic organism and used with or adapted for use with this invention.

As used herein, the terms "protease" and "proteinase" are synonymous and refer to an enzyme that has proteolytic activity, for example, catalyzes the cleavage of peptide bonds, for example, in proteins, polypeptides, oligopeptides, and peptides (collectively "peptides"). As used herein, the terms "thermophilic protease" and "thermophilic proteinase" are synonymous, and refer to a protease having optimal proteolytic activity at elevated temperatures, for example, at or above about 65°C, or from about 65°C to about 90°C, and includes proteases isolated from thermophilic organisms and proteases isolated from organisms other than thermophilic organisms that have optimal proteolytic activity as described above, including any variants of such proteases such as those that have subsequently been modified or engineered. Thermophilic proteases particularly suitable for use in the present invention include those that are heat denaturable and/or exhibit autolytic activity at or above about 90°C, and are preferably permanently heat-inactivated at elevated temperatures, for example, at temperatures at or above about 90°C.

Other characteristics of particularly suitable thermophilic proteases are activity over a wide pH range, and activity in a broad variety of conditions. Hence, particularly suitable proteases can be used with most buffers and so will function in buffers particularly suited for the mesophilic enzyme.

Exemplary thermophilic proteases particularly suitable for use in the methods of the present invention include EA1 proteinase, a neutral proteinase isolated from *Bacillus* species strain EA1, and variants thereof, and Ak1 proteinase, a serine protease isolated from *Bacillus* species strain Ak1, and variants thereof.

The isolation and sequence of the gene encoding EA1 and the recombinant expression of the proteinase encoded thereby is described in Saul et al., "Sequence of the gene encoding a highly thermostable neutral proteinase from Bacillus sp. strain EA1: expression in Escherichia coli and characterisation." Biochemica et Biophysica Acta 1308; 74-80 (1996). The sequence encoding native EA1 is available under Genbank Accession No. U25630, and the amino acid sequence of the native protein is available under Genbank Accession No. AAB 18653.

The isolation and sequence of the gene encoding Ak1 and the recombinant expression of the proteinase encoded thereby is described in Maciver B, et al., "Cloning and sequencing of a serine proteinase gene from a thermophilic Bacillus species and its expression in Escherichia coli." Appl Environ Microbiol. 60(11): 3981-3988 (1994). The sequence encoding native Ak1 is available under Genbank Accession No. L29506, and the amino acid sequence of the native protein is available under Genbank Accession No. AAA63688.

Further examples of thermophilic proteases suitable for use in the invention include *Thermus* spp. proteases, including for example, *Thermus* sp. Rt41A protease (see Munro G, et al., "A gene encoding a thermophilic alkaline serine proteinase from Thermus sp. strain Rt41A and its expression in Escherichia coli." Microbiology 141; 1731-1738 (1995), and Genbank Accession No. AAA82980, aqualysin I (see Terada I, et al., "Unique precursor structure of an extracellular protease, aqualysin I, with NH2- and COOH-terminal pro-sequences and its processing in Escherichia coli." J Biol Chem 265:6576-6581 (1990) and Genbank Accession No. BAA14135, Caldolysin (see D.A. Cowan and R.M. Daniel, "Purification and some properties of an extracellular protease (Caldolysin) from an extreme thermophile." Biochimica et Biophysica Acta, 705: 293-305 (1982)), Caldolase (see Saravani A, et al., "Caldolase: an extracellular serine protease from a Thermus spp." Biochemical Journal 262: 409-416 (1989)), *Thermus* sp. strain Rt4A2 protease (see Freeman SA, et al., "Characterisation of a chelator-resistant proteinase from Thermus strain Rt4A2." Biochemical Journal. 295: 463-469 (1993) and Genbank Accession No. AAB28684), and the alkaline serine proteases discussed in Munro G, et al., above.

Yet further examples of thermophilic proteases include members of the thermolysin family of neutral proteinases, such as for example, *Bacillus caldolyticus* YP-T protease (see van den Burg B, et al., "A highly thermostable neutral protease from Bacillus caldolyticus: cloning and expression of the gene in Bacillus subtilis and characterization of the gene product." J. Bacteriol. 173: 4107-4115 (1991), and Genbank Accession No. AAA22623), BT1 (see Vecerek B and Kyslik P, "Cloning and sequencing of the neutral protease-encoding gene from a thermophilic strain of Bacillus sp." Gene 158: 147-148 (1995), and Genbank Accession No. AAC43402), *Bacillus stearothermophilus* NprT protease (see Tagaki M, et al., "Nucleotide sequence and promoter region for the neutral protease gene from Bacillus stearothermophilus." J Bacteriol. 163: 824-831 (1985) and Genbank Accession No. AAA22621), *Bacillus stearothermophilus* NprM protease (see Kubo M and Imanaka T, "Cloning and nucleotide sequence of the highly thermostable neutral protease gene from Bacillus stearothermophilus." J Gen Microbiol 134: 1883-1892 (1988) and Genbank Accession No. AAB02774), and *Bacillus stearothermophilus* NprS protease (see Nishiya Y and Imanaka T, "Cloning and nucleotide sequences of the Bacillus stearothermophilus neutral protease gene and its transcriptional activator gene." J. Bacteriol. 172: 4861-4869 (1990), and Genbank Accession No. AAA22625), and suitable proteases identified in Coolbear T, et al., "Screening of strains identified as extremely thermophilic bacilli for extracellular proteolytic activity and general properties of the proteinases from two of the isolates." J. Applied Bacteriology 71: 252-264 (1991).

It will be appreciated that the use and/or inactivation (whether reversible or irreversible) of proteases exhibiting high stability at high temperatures, for example the *Thermus* proteases such as Caldolysin, Caldolase, and RT4A2 and the like, can require different conditions than those used for proteases exhibiting less stability at high temperature, such as for example EA1. For example, irreversible inactivation of such proteases exhibiting high stability at high temperatures may require very low calcium concentrations, the addition of chelators, and the like as described herein in combination with incubation at high temperature.

Methods for the modification of a thermophilic protease so as to alter the characteristics including structural or functional characteristics of the protease are well known in the art. See, for example, Eijsink VG, et al., "Structural determinants of the stability of thermolysin-like proteinases." Nat Struct Biol. 2(5):374-9 (1995). Such modification, including for example amino acid substitution and/or post translational modification, may be directed to modification of the functional characteristics of the protease, for example, the thermostability, proteolytic activity, and/or autolytic activity at a given temperature or temperature range, the substrate specificity, the dependence upon one or more co-factors, the pH dependence, and the like. For example, a variant thermophilic protease may be rendered more suitable to use in the invention than the native protease by lowering or raising the temperature at which it becomes susceptible to autolysis.

The term "variant" with reference to polypeptides encompasses naturally occurring, recombinantly and synthetically produced polypeptides. Variant polypeptide sequences preferably exhibit at least 50%, more preferably at least 70%, more preferably at least 80%, more preferably at least 90%, more preferably at least 95%, more preferably at least 98%, and most preferably at least 99% identity to the specified sequences. Identity is found over a comparison window of at least 20 amino acid positions, preferably at least 50 amino acid positions, more preferably at least 100 amino acid positions, and most preferably over the entire length of a polypeptide.

Polypeptide sequence identity can be determined in the following manner. The subject polypeptide sequence is compared to a candidate polypeptide sequence using BLASTP (from the BLAST suite of programs, version 2.2.10 [Oct 2004]) in b12seq, which is publicly available from NCBI (ftp://ftp.ncbi.nih.gov/blast/). The default parameters of b12seq are utilized except that filtering of low complexity regions should be turned off.

Polypeptide sequence identity may also be calculated over the entire length of the overlap between a candidate and subject polynucleotide sequences using global sequence alignment programs. EMBOSS-needle (available at http:/www.ebi.ac.uk/emboss/align/) and GAP (Huang, X. (1994) On Global Sequence Alignment. Computer Applications in the Biosciences 10, 227-235.) as discussed above are also suitable global sequence alignment programs for calculating polypeptide sequence identity.

Use of BLASTP as described above is preferred for use in the determination of polypeptide variants.

Polypeptide variants also encompass those which exhibit a similarity to one or more of the specifically identified sequences that is likely to preserve the functional equivalence of those sequences and which could not reasonably be expected to have occurred by random chance. Such sequence similarity with respect to polypeptides may be determined using the publicly available b12seq program from the BLAST suite of programs (version 2.2.10 [Oct 2004]) from NCBI (ftp://ftp.ncbi.nih.gov/blast/). The similarity of polypeptide sequences may be examined using the following UNIX command line parameters:
b12seq -i peptideseq1 -j peptideseq2 -F F -p blastp

Variant polypeptide sequences preferably exhibit an E value of less than 1 x 10⁻⁵, more preferably less than 1 x 10 ⁻⁶, more preferably less than 1 x 10 ⁻⁹, more preferably less than 1 x 10 ⁻¹², more preferably less than 1 x 10 ⁻¹⁵, more preferably less than 1 x 10 ⁻¹⁸ and most preferably less than 1 x 10 ⁻²¹ when compared with any one of the specifically identified sequences.

The parameter -F F turns off filtering of low complexity sections. The parameter -p selects the appropriate algorithm for the pair of sequences. This program finds regions of similarity between the sequences and for each such region reports an "E value" which is the expected number of times one could expect to see such a match by chance in a database of a fixed reference size containing random sequences. For small E values, much less than one, this is approximately the probability of such a random match.

Conservative substitutions of one or several amino acids of a described polypeptide sequence without significantly altering its biological activity are also included. A skilled artisan will be aware of methods for making phenotypically silent amino acid substitutions (see, e.g., Bowie et al., 1990, Science 247, 1306).

As used herein, the term "contaminant" includes any undesired matter, including components in or on a composition whose continued presence in or on said composition is or becomes undesirable, and any undesired activity including, for example, enzymatic activity, that is or becomes undesirable. For example, in an exemplary method of the invention, in a first step an undesired enzyme substrate present in a composition may be considered a contaminant, while in a subsequent step the continued activity of said enzyme, being no longer desirable, may be considered a contaminant.

It will further be appreciated that removal of contamination can be effected in or on any composition in or on which conditions amenable to the enzymatic activity of one or more mesophilic enzyme and a thermophilic protease can be maintained. Such compositions include samples such as biological samples, solutions, solid surfaces, containers, and the like.

Clearly, in the context of the preparation of compositions for use in subsequent analytic or diagnostic procedures, including the preparation of samples or solutions for use in such procedures, the removal of contaminants offers the prospect of more sensitive, reproducible, and robust analyses or diagnoses, provided such removal has minimal detrimental effect of its own.

Many biological substances, especially nucleic acids, present special challenges in terms of isolating them from their natural environment and their subsequent handling and analysis. They are often present in very small concentrations, and are often found in the presence of many other solid and dissolved substances. In addition to these contaminants, extraneous contaminants are frequently present in solutions and compositions used in the processing and analysis of said samples.

Contamination is a significant problem in most isolation, preparative, analytic and diagnostic methods, and particularly so where such methods are sensitive. For example, a number of molecular biological techniques are highly sensitive to contamination, which may lead to inaccurate results, for example, a false positive or a false negative result. For example, PCR techniques are capable of yielding a significant multiplication of target molecules, and are thus highly sensitive to the presence of contaminating molecules, even where pure test organisms and purportedly ultra-pure working solutions are used. Contaminating nucleic acid from contaminating organisms remains even though the organisms may be dead and the solutions sterile.

Contamination of the sample and/or working solutions may occur simply as a result of the reagent tubes being opened to the atmosphere or being touched by a technician. In addition, contamination may arise during preparation of the working solutions, or may remain attached to containers, equipment, glassware and the like used in the preparation or storage of samples and working solutions. For example, traces of nucleic acid may remain attached to glassware even after acid wash treatments.

Such contamination renders more problematic the isolation, handling, and analysis of such samples, such as, for example, in biospecific assays which allow the detection of specific analytes or specific analyte properties.

Examples of biospecific assays are hybridisation assays, immuno assays and receptor-ligand assays. Hybridisation assays use the specific base-pairing for the molecular detection of nucleic acid analytes e.g. RNA, and DNA. Hence, oligonucleotide probes with a length of 18 to 20 nucleotides may enable the specific recognition of a selected complementary sequence e.g. in the human genome. Another assay which entails the selective binding of two oligonucleotide primers is the polymerase chain reaction (PCR) described in U.S. Pat. No. 4,683,195. PCR is a standard diagnostic procedure. This procedure allows the selective amplification of a specific nucleic acid region to detectable levels by a thermostable polymerase in the presence of desoxynucleotide triphosphates in multiple cycles. The present disclosure is in one embodiment directed to improvements on an existing procedure to minimise contamination of compositions, including working solutions, from nucleic acid or nucleic acid-degrading enzymes, including those from contaminating organisms and from treatment practices.

Whilst this discussion is provided with reference to PCR techniques, it should be appreciated the discussion is not to be seen as limiting the scope of the invention. The invention has application with a range of procedures, including diagnostic and other nucleic acid techniques where contamination, including for example nucleic acid and/or nucleic acid-modifying enzymes in compositions, such as for example working solutions, may detrimentally impact on results derived therefrom or on the complexity of the procedures.

For ease of reference throughout the specification, the term "nucleic acid" should be taken to include DNA (e.g., cDNA or genomic DNA), RNA (e.g., an mRNA) and analogs of the DNA or RNA. A DNA or RNA analog can be synthesized from nucleotide analogs. The nucleic acid can be a single-stranded or double-stranded molecule, and may be a heteroduplex or otherwise comprise more than one nucleic acid. Further, the nucleic acid of the present invention may comprise a modified backbone and/or modified bases, and includes the component moieties of nucleic acid, including nucleosides, nucleotides and phosphates and sugars thereof. A variety of modifications to nucleic acid are known in the art for multiple useful purposes. The term "nucleic acid" as it is employed herein embraces such chemically, enzymatically or metabolically modified forms of nucleic acid, as well as the chemical forms of DNA and RNA characteristic of viruses and cells, including simple and complex cells.

PCR is a highly sensitive technique, and can be used to amplify the number of copies of a specific region of nucleic acid, often from minute quantities of starting material, in order to produce sufficient nucleic acid for analysis. A number of generally applicable steps are involved in PCR procedures, involving preparation of the sample, the master mix of reagents and the oligonucleotide primers. Therefore, there are a number of stages where contamination of the PCR can occur. PCR techniques have for many years been hindered by contamination.

Such contamination may occur not only from contaminating nucleic acid in the preparation of working solutions, but also simply via handling or exposure of the solutions to the air during subsequent steps of the PCR procedure. Even with purportedly pure reagents, contaminating nucleic acid can still be a problem. Results from standard PCR techniques must take account of such contamination in the reporting of results.

For example, contaminating DNA is a particular problem in forensic science, where human DNA contamination can result in a failed conviction, and in bacterial diagnostics where trace bacterial DNA is ever present in low concentrations in reagents. Despite using ultra-pure reagents, the problem still requires a reduction in PCR cycle numbers. Typically, microbial geneticists working with 16S rRNA genes must keep PCR cycle numbers below 30 (preferably 25) and forensic scientists below 30-35. The cost is a loss of sensitivity.

For many diagnostic techniques, current protocols require treating all equipment, glassware and reagents with undesirable chemicals prior to use to remove contamination. For example, most protocols involving RNA analysis requires all equipment, glassware and reagents be treated with the toxic chemical diethylpyrocarbonate (DEPC) prior to use to remove ribonucleases. Frequently, equipment and reagents must be set aside and dedicated for this use alone, or the use of large quantities of disposable equipment is required.

The methods of the present invention have numerous advantages over current techniques, shortening the amount of time required for the preparation of compositions including reagents and working solutions, and ensuring they are free from contamination, for example, by nucleic acid and/or nucleic acid-degrading enzymes.

Moreover, the methods of the present invention, reliant as they are on the temperature at which a composition is maintained for the initiation and termination of enzymatic or proteolytic activity, rather than the sequential addition and removal of different activities to the composition, are readily automatable, using equipment well know in the art. For example, thermal cyclers commonly used in PCR techniques are particularly suited for use in the methods of the invention. Robotic equipment commonly used in the laboratory is also well suited to use in the methods of the present invention. For example, robotic workstations including robotic liquid handling workstations such as the Biomek 2000 Workstation from Beckman Coulter, the Tecan Genesis liquid handler, the Cartesian Technologies PixSys 4200 SynQuad liquid handler, the Eppendorf epMotion 5070 workstation, the Qiagen Biorobot M96, and the like, can easily be combined with other automatable equipment, including plate sealers and robotic arms (such as those available from Beckman Coulter) and the abovementioned thermal cycling equipment to automate the methods of the invention.

It is envisaged the present invention will provide a new standard for purification for critical applications, such as forensic and evidential applications, where contamination-free reagents are critical.

The procedure of one embodiment of the present invention requires that containers, reagents and working solutions be taken before use and treated with at least one nucleic acid-degrading enzyme to minimise contaminating nucleic acid, and at least one thermophilic protease. Either or both mesophilic and thermophilic nucleic acid-degrading enzymes are suitable for use. Typically, nucleases are used to degrade contaminating nucleic acid.

The containers, reagents and working solutions may be stored at room temperature or a desired optimum temperature until required. The incubation period is dictated only by the period required to effect degradation of the contamination, for example the contaminating nucleic acid, and the time until the containers, reagents and working solutions are required for use. Accordingly, the containers, reagents and working solutions may be incubated at room temperature for any variable time.

The at least one nucleic acid-degrading enzyme must be active and stable at or below about 65°C. The at least one nucleic acid-degrading enzyme must also be capable of being proteolytically degraded or irreversibly denatured at about 65°C to about 90°C.

Alternatively, the containers, reagents and working solutions may then be apportioned and frozen until needed, to ensure stability of the nucleic acid-degrading enzyme and protease mixture, then maintained at a temperature at which the one or more nucleic acid-degrading enzyme has enzymatic activity to effect decontamination. This temperature will be dependent on the particular one or more nucleic acid-degrading enzyme used, for example, whether a mesophilic or thermophilic nucleic acid-degrading enzyme or both is used.

The one or more nucleic acid-degrading enzyme used in the initial decontamination step will also degrade any sample nucleic acid template it is brought into contact with, so therefore it too is a contaminant and it's nucleic acid-degradative activity must be removed before subsequent use, for example, in PCR. While many nucleic acid-degrading enzymes are capable of being inactivated by denaturation, frequently they are also capable of subsequently refolding into an active form. Clearly this is undesirable as it can result in degradation of sample and/or inaccurate or inefficient analysis, for example, degradation of sample template and reduced efficiency of a PCR procedure.

It is therefore preferred that the nucleic acid-degrading enzyme be degraded. Accordingly, the containers, reagents and working solutions may be heated to from about 65°C to about 90°C, at which temperature the thermophilic protease exhibits proteolytic activity, thereby degrading and irreversibly inactivating the one or more nucleic acid-degrading enzyme. Thus, the one or more nucleic acid-degrading enzyme cannot then refold to become active again.

Preferably a thermophilic protease is also used in the initial decontamination step to ensure degradation of the nuclease and any other contaminating enzymes.

The key requirements for the protease in this invention are that it has sufficient stability to be active somewhere in the temperature range of about 65°C to about 90°C, but not so much stability that it is difficult to denature and/or autolyse at or above about 90°C, and that it has a temperature-activity profile such that it has no or low activity below about 40°C, preferably below about 45°C, below about 50°C, below about 55°C, more preferably below about 60°C, more preferably below about 65°C.

By using a suitable thermophilic protease, the containers, reagents and solutions may be maintained at from about 65°C to about 90°C to rapidly degrade the one or more nucleic acid-degrading enzymes. As the protease itself may interfere with the subsequent use, particularly those steps reliant on a proteinaceous component such as an enzyme, for example a polymerase in a PCR, and the accuracy of the results obtained therefrom, the protease must also be degraded. For suitable proteases, heating the solutions to about 90°C or above will cause rapid self-degradation such as autolysis and/or denaturation of the protease, resulting in irreversible inactivation of the protease.

Accordingly, the containers, reagents and working solutions are thereby substantially purified for use, for example for use in ensuing PCR procedures, and the likelihood of erroneous results from contaminating nucleic acid and/or nucleic acid degrading enzymes is minimised.

Having a closed-tube system of purification prevents exposure of the reagents to the air, and reduces the number of steps where opening and closing the reagent tubes is required, thus minimising any possible contamination.

It is envisaged that one embodiment of the present invention may set a new standard for reliability for evidential and other critical applications of PCR and other molecular biology diagnostic techniques through the provision of an ability to remove from compositions any contamination from nucleic acid and/or nucleic acid degrading enzymes.

The present invention has been described with reference to either or both mesophilic and thermophilic nucleic acid degrading enzymes, mesophilic enzymes, and thermophilic proteases. However, it should be appreciated that variations to the present invention are possible and will fall within the scope of the present invention as herein described.

With reference to Figure 1 and discussion herein, in a preferred aspect there is provided a method for purifying working solutions for Polymerase Chain Reaction (PCR) techniques.

The method involves taking one or more PCR working solution and adding to the solution, before any PCR is undertaken, one or more mesophilic nuclease. The nuclease is added to degrade nucleic acid contamination present in the solution, such as contamination from an external source.

Preferably at least one thermophilic protease is added, preferably concurrently with the nuclease.

In order to effect decontamination of said one or more solution, the solution is maintained at a desired temperature for nucleolytic activity of said one or more nuclease at or below about 65°C, and preferably at the optimum temperature for nucleolytic activity, until required for use. There is no maximum time limit for this maintenance other than ensuring the activity of the one or more nuclease and the at least one protease is maintained. The minimum time is dictated only by the need to ensure the contaminating nucleic acid is degraded.

To complete the purification procedure prior to using the one or more solution, first the one or more nuclease, then the protease must be removed. The solution is subjected to at least one heat treatment step, wherein the solution is maintained at a temperature of between about 65°C and about 90°C.

This heat treatment step substantially denatures the nuclease to preclude degradation of the sample nucleic acid. In embodiments in which at least one thermophilic protease is employed, said thermophilic protease is active at this temperature, and degrades the nuclease and any other contaminating proteins, including enzymes.

Maintaining the one or more solution at about 90°C or above will then result in rapid self-degradation such as autolysis and/or denaturation of the proteases.

The working solutions are then allowed to cool and may be then used in standard PCR and/or other molecular biology diagnostic procedures.

The method is characterised by the use of a nuclease to remove existing nucleic acid contamination, followed by a heat treatment step to denature said nuclease thereby to reduce nuclease-dependent degradation of the sample nucleic acid in the PCR.

Another aspect of the method involves using a thermophilic protease to degrade the nuclease and any other contaminating proteins including enzymes, followed by a heat treatment step to degrade and/or denature said thermophilic protease.

The invention consists in the foregoing and also envisages constructions of which the following gives examples only.

### EXAMPLES

### EXAMPLE 1: Removal of DNA from solutions.

This example describes the use of cocktails of mesophilic nuclease, such as DNAsel, in conjunction with thermophilic protease to remove contaminating DNA from solutions.

### Methods

PCR reagents and solutions were deliberately contaminated with a high molar quantity of target DNA (5 ng). The reagents and solutions were treated with a DNAsel/ EA1 thermophilic protease cocktail as follows: 37°C for 15 min, 75°C for 15 min, and 95°C for 15 min.

To test for residual DNase1 activity, the template and oligonucleotides were incubated at 37°C for an additional hour. Reagents and solutions with DNAse1 added but lacking EA1 thermophilic protease were used as controls for the removal of DNAse1 activity.

### Results

The combination of mesophilic nuclease and thermophilic protease was highly successful in removing DNA contamination from PCR reagents and solutions. The gel image depicted in Figure 2 demonstrates the efficacy of removal of DNA from deliberately contaminated PCR reagents. Even after 40 cycles of PCR, no evidence of product from the contaminating DNA is visible (see Figure 2, lanes 12 and 13).

To verify that the lack of product was not the result of residual DNAse1 activity, the cleaned reagents were used in a subsequent PCR, see Figure 3. Here, when DNAse1 alone is used and taken through the full heat treatment, residual activity remains which appears to be degrading the primers. Clearly, addition of the thermophilic protease is effective to remove DNAse1 activity, allowing the production of PCR product (see Figure 3, lanes 6 and 7).

### Conclusions

This method can be used to remove undesirable enzyme activities in solutions where a simple heat treatment is ineffective. The technique effectively makes thermostable enzymes heat-labile.

### EXAMPLE 2: Removal of contaminants from solutions.

This example describes the use of a combination of mesophilic nuclease, such as DNAsel, in conjunction with thermophilic protease to remove contamination from solutions.

### Methods

Reagents and solutions were deliberately contaminated with a high molar quantity of target DNA (5 ng). The reagents and solutions were treated with a DNAsel/ EA1 thermophilic protease combination at 37°C for 15 min, followed by 75°C for 15 min.

To test for residual DNase1 activity, the template and oligonucleotides were incubated at 37°C for an additional hour. Reagents and solutions with DNAse1 added but lacking EA1 thermophilic protease were used as controls for the removal of DNAse1 activity.

### Results

The combination of mesophilic nuclease and thermophilic protease was highly successful in removing contamination, both nucleic acid and added mesophilic enzyme, from reagents and solutions.

### Conclusions

This method can be used to remove undesirable enzyme activities in solutions where a simple heat treatment is ineffective. The technique effectively makes thermostable enzymes heat-labile. By omitting the incubation at 95C (as performed in Example 1 above to irreversibly inactivate the thermophilic protease), the activity of the thermophilic protease is preserved for subsequent use.

### EXAMPLE 3: Removal of heat resistant enzymes.

This example describes the use of thermophilic protease to remove contaminating heat resistant mesophilic enzymes from solutions.

### Methods

Samples of *Pvu*II were treated as follows: control samples were subjected to no further treatment; heat treated *Pvu*II was incubated at 75°C for 15 min; EAl-treated *Pvu*II was incubated at 75°C for 15 min in the presence of EA1 thermophilic protease. These samples of *Pvu*II was then added to aliquots of plasmid DNA and incubated. Samples were then visualised by gel electrophoresis and UV transillumination.

### Results

The restriction endonuclease *Pvu*II is resistant to heat inactivation. This is clearly shown in Figure 5, where viable enzymatic activity is observed even after heat treatment at 75°C for 15 min (Figure 4, lanes 4 and 5). Incubation at 75°C in the presence of thermophilic protease results in the complete removal of enzymatic activity (Figure 4, lanes 6 and 7).

### Conclusions

This method can be used to remove undesirable enzyme activities in solutions where a simple heat treatment is ineffective. The technique effectively makes thermostable enzymes heat-labile. Following removal of restriction enzymatic activity, digested DNA is immediately available for subsequent use, such as ligation, with the protease in place. At normal ligation temperatures (20°C and below) the protease has no measurable activity.

### EXANIPLE 4: Removal of starch from cloth.

This example describes the use of thermophilic protease to remove mesophilic enzyme from solid substrate, itself used to remove contamination from the solid substrate.

### Methods

Starched cloth is de-sized using a mesophilic amylase in combination with a thermophilic protease. The cloth is maintained at approximately 45°C (although any temperature in which the amylase is active, such as from 20°C to 60°C, is suitable) for 2 hours, then the temperature is increased to approximately 75°C (from 65°C to 90°C is also suitable). The cloth is maintained at this temperature for 1 hour, then the temperature is again raised to 95°C for 1 hour.

### Results

The incubation at 45°C is sufficient to remove starch from the cloth, through the action of the amylase. Incubation at 75°C activates the protease, degrading the amylase. Finally, incubation at 95°C initiates autolysis of the protease.

### Conclusions

This method allows the removal of an unwanted contaminant, in this case starch, from a solid substrate. Advantageously, the method also allows the removal of both the enzyme used to effect starch removal, and the protease effecting such removal, resulting in de-sized cloth with very low residual protein content.

### EXAMPLE 5: Removal of ethanol from solution.

This example describes the use of thermophilic protease to remove mesophilic enzyme, itself used to remove contamination from solution.

### Methods

Unwanted ethanol is removed from a solution using ethanol dehydrogenase and/or alcohol oxidase in combination with a thermophilic protease. The solution is maintained at approximately 37°C (although any temperature in which the mesophilic enzyme is active, such as from 20°C to 50°C, is suitable) for 1 hour, then the temperature is increased to approximately 75°C (from 65°C to 90°C is also suitable). The solution is maintained at this temperature for 1 hour, then the temperature is again raised to 95°C for 1 hour.

### Results

The incubation at 37°C is sufficient to remove ethanol from the solution, through the action of the ethanol dehydrogenase and/or alcohol oxidase. Incubation at 75°C activates the protease, degrading the mesophilic enzyme. Finally, incubation at 95°C initiates autolysis of the protease.

### Conclusions

This method allows the removal of an unwanted contaminant, in this case ethanol, from a solution. Advantageously, the method also allows the removal of both the enzyme(s) used to effect ethanol removal, and the protease effecting such removal, resulting in a solution with no contaminating ethanol, and with very low residual protein content.

### EXAMPLE 6: Decontamination of sample container.

This example describes the use of thermophilic protease to remove mesophilic enzyme itself used to remove contamination from the solid substrate of a sample collection container.

### Methods

A sample collection container, in this case an eppendorf tube fitted with a valved screw cap lid, to be used for the collection of solid sample is decontaminated as follows. A solution comprising DNAseI and a thermophilic protease is added to the eppendorf tube, and vortexed to completely cover the inner surface of the tube. The tube is maintained at approximately 40°C (although any temperature in which the DNAseI is active, such as from 20°C to 60°C, is suitable) for 2 hours, with frequent vortexing to distribute the solution over the inner surface. The temperature is then increased to approximately 75°C (from 65°C to 90°C is also suitable). The tube is maintained at this temperature for 1 hour, then the temperature is raised to 95°C for approximately 15 min. Finally, the tube is incubated at 100°C for 30min.

### Results

The incubation at 40°C is sufficient to remove any DNA contamination from the tube, through the action of the exonuclease. Incubation at 75°C activates the protease leading to the degradation of the nuclease, whereupon incubation at 95°C initiates autolysis of the protease. Finally, the incubation at 100°C allow the solution to boil off through the valved lid, resulting in a dry tube free of DNA contamination.

### Conclusions

This method allows the removal of an unwanted contaminant, in this case DNA, from the solid surface of a sample collection tube. Advantageously, the method also allows the removal of both the enzyme used to effect DNA removal, and the protease effecting such removal. The use of a tube with a valved top allows the removal of liquid by boiling, resulting in a dry tube free of DNA contamination and with very low residual protein content, suitable for the collection and storage of solid samples, for example, for later forensic analysis.

### EXAMPLE 7: Removal of lipid from sample solution.

This example describes the use of thermophilic protease to remove mesophilic enzyme, itself used to remove contamination from solution.

### Methods

Unwanted lipid is removed from a sample solution using lipase in combination with a thermophilic protease. The solution is maintained at approximately 37°C (although any temperature in which the lipase is active, such as from 20°C to 50°C, is suitable) for 1 hour, then the temperature is increased to approximately 75°C (from 65°C to 90°C is also suitable). The solution is maintained at this temperature for 1 hour, then the temperature is again raised to 95°C for 1 hour.

### Results

The incubation at 37°C is sufficient to remove lipid from the sample, through the action of the lipase. Incubation at 75°C activates the protease, degrading the lipase. Finally, incubation at 95°C initiates autolysis of the protease.

### Conclusions

This method allows the removal of an unwanted contaminant, in this case lipid, from a sample solution. Advantageously, the method also allows the removal of both the enzyme(s) used to effect lipid removal, and the protease effecting such removal, resulting in a sample with no contaminating lipid, and with very low residual protein content.

The above methods enable two-step reactions to occur in a closed tube thermocycler controlled format. This creates the opportunity to combine a variety of sample and/or DNA modification steps, which ends with a cleaned DNA composition, free of undesired enzymatic activity ready for the next step of analysis. The approach removes the need for solvent extractions and ethanol precipitations. Furthermore, the fact that contamination, such as undesired enzymatic activity, can be removed without the necessity of addition or removal of further components to the composition with the attendant exposure of the composition to potential contamination from exogenous sources, renders the methods of the invention highly attractive as a means to develop robust analytic and diagnostic procedures, particularly those that are sensitive to contamination. For example, microbial identification and monitoring, plant tissue analysis, forensic DNA analysis and any other technique dealing with either trace DNA and/or many samples that need automated handling are particularly suited to use with methods of the invention.

It is not the intention to limit the scope of the invention to the abovementioned examples only. As would be appreciated by a skilled person in the art, many variations are possible without departing from the scope of the invention (as set out in the accompanying claims).

## Claims

1. The use of at least one thermophilic protease and one or more mesophilic enzyme in combination in the removal of at least one contaminant from a composition, wherein the at least one thermophilic protease has optimal proteolytic activity at or above 65°C and the one or more mesophilic enzyme has optimal enzymatic activity below 65°C, wherein said contaminant is a substrate of one or more mesophilic enzyme, wherein the at least one thermophilic protease exhibits proteolytic activity towards the one or more mesophilic enzyme, and wherein said composition is not a nucleic acid sample comprising nucleic acid for testing.

2. The use of claim 1 wherein said at least one thermophilic protease is selected from the group consisting offal, Ak1, NprS, NprT, BT1, YP-T, aqualysin I, NprM, Caldolysin, Caldolase, Rt41 A, RT4A2, and variants thereof having optimal proteolytic activity at or above 65°C.

3. The use of claim 2 wherein said thermophilic protease is EA1 and/or Ak1 or variants thereof.

4. The use of any one of claims 1 to 3 wherein said one or more mesophilic enzyme is a nucleic acid-modifying enzyme.

5. The use of claim 4 wherein said nucleic acid-modifying enzyme is selected from the group comprising nucleases, phosphatases, kinases, polymerases, synthetases, ligases, methylases, transferases and recombinases.

6. The use of any one of claims 1 to 3 wherein said one or more mesophilic enzyme is selected from the group consisting of ATPases, cytochromes, deaminases, dehydrogenases, decarboxylases, desaturases, dioxygenases, elastases, endopeptidases, flavoproteins, flippases, hydrogenases, hydrolases, hydroxylases, integrases, isomerases, kinases, ligases, lipases, lipoxygenases, lyases, mutases, oxidases, oxioreductases, peptidases and proteolytic enzymes, permeases, peroxidases, phosphorylases, recombinases, reductases, regulatory enzymes, ribonucleases, sulfatases, synthases, synthetases, telomerases, and transferases.

7. The use of any one of claims 1 to 6 wherein said composition is to be used in a polymerase chain reaction.

8. A method for the removal of at least one contaminant from a composition, wherein said contaminant is a substrate of one or more mesophilic enzyme, said method comprising the steps of:
adding to said composition said one or more mesophilic enzyme and at least one thermophilic protease; and
maintaining said composition at a first temperature or temperature range at which said one or more mesophilic enzyme has enzymatic activity, but at which said at least one thermophilic protease has minimal or no proteolytic activity; and
maintaining said composition at a second temperature or temperature range at which said at least one thermophilic protease has proteolytic activity; and
maintaining said composition at a temperature suitable for use;
wherein said one or more mesophilic enzyme does not exhibit activity effective to remove cell walls.

9. The method of claim 8 wherein said method comprises after said maintenance at a second temperature or temperature range one or more repetitions of the additional steps of:
adding to said composition one or more mesophilic enzyme; and
maintaining said composition at a temperature or temperature range at which said one or more mesophilic enzyme has enzymatic activity, but at which said at least one thermophilic protease has minimal or no proteolytic activity; and
maintaining said composition at a temperature or temperature range at which said at least one thermophilic protease has proteolytic activity.

10. The method of claim 8 or claim 9 wherein said method comprises the additional step of maintaining said composition at a third temperature or temperature range sufficient to cause denaturation and/or autolysis of said at least one thermophilic protease.

11. The method of any one of claims 8 to 10 wherein said first temperature or temperature range is between about 10°C and about 65°C.

12. The method of any one of claims 8 to 11 wherein said first temperature or temperature range is between about 20°C and about 50°C.

13. The method of any one of claims 8 to 12 wherein said first temperature or temperature range is between about 30°C and about 40°C.

14. The method of any one of claims 8 to 12 wherein said first temperature or temperature range is between about 40°C and about 65°C.

15. The method of any one of claims 8-10, 12 and 13 wherein said second temperature or temperature range is between about 65°C and about 85°C.

16. The method of claim 15 wherein said second temperature or temperature range is between about 65°C and about 80°C.

17. The method of any one of claims 8 to 16 wherein said second temperature or temperature range is between about 70°C and about 80°C.

18. The method of any one of claims 8 to 17 wherein said second temperature is about 75°C.

19. The method of claim 10 wherein said third temperature or temperature range is between about 90°C and about 105°C.

20. The method of claim 19 wherein said third temperature or temperature range is between about 90°C and about 100°C.

21. The method of claim 19 wherein said third temperature or temperature range is between about 90°C and about 95°C.

22. The method of claim 21 wherein said third temperature is about 95°C.

23. The method of any one of claims 8 to 22 wherein said one or more mesophilic enzyme is a nucleic acid-modifying enzyme.

24. The method of claim 23 wherein said enzyme is selected from the group consisting of nucleases, phosphatases, kinases, polymerases, synthetases, ligases, methylases, transferases and recombinases.

25. The method of any one of claims 8 to 22 wherein said one or more mesophilic enzyme is selected from the group consisting of ATPases, cytochromes, deaminases, dehydrogenases, decarboxylases, desaturases, dioxygenases, elastases, endopeptidases, flavoproteins, flippases, hydrogenases, hydrolases, hydroxylases, integrases, isomerases, kinases, ligases, lipases, lipoxygenases, lyases, mutases, oxidases, oxioreductases, peptidases and proteolytic enzymes, permeases, peroxidases, phosphorylases, recombinases, reductases, regulatory enzymes, ribonucleases, sulfatases, synthases, synthetases, telomerases, and transferases.

26. The method of any one of claims 8 to 25 wherein said composition is or is in a closable container.

27. The method of claim 26 wherein said container remains closed after addition of said one or more mesophilic enzyme and said at least one thermophilic protease and prior to use.

28. The method of any one of claims 8 to 27 wherein said method is performed in a closed tube system.

29. The method of any one of claims 8 to 28 wherein said method is automated.

30. The method of claim 29 wherein said method utilises a thermal cycler.

31. The method of claim 30 wherein said method utilises a thermal cycler in combination with robotic liquid handling.

32. The method of any one of claims 8 to 31 wherein said at least one thermophilic protease is selected from the group consisting of EA1, Ak1, NprS, NprT, BT1, YP-T, aqualysin I, NprM, Caldolysin, Caldolase,Rt41A, RT4A2, and variants thereof.

33. The method of claim 32 wherein said thermophilic protease is EA1 and/or Ak1 or variants thereof.

34. A method for the removal of nucleic acid from a composition, said method comprising the steps of:
adding to said composition one or more mesophilic nucleic acid-degrading enzyme and at least one thermophilic protease; and
maintaining said composition at a first temperature or temperature range at which said one or more mesophilic nucleic acid-degrading enzyme has enzymatic activity, but at which said at least one thermophilic protease has minimal or no proteolytic activity; and
maintaining said composition at a second temperature or temperature range at which said at least one thermophilic protease has proteolytic activity; and
maintaining said composition at a temperature suitable for use.

35. The method of claim 34 wherein said one or more mesophilic nucleic acid-degrading enzyme is a nuclease.

36. The method of any one of claims 34 or 35 wherein said composition is for use in a PCR procedure.

37. A method for purifying one or more working solution for nucleic acid diagnostic techniques in a closed-tube system, said method comprising the steps of:
adding to said one or more working solution one or more mesophilic nuclease and at least one thermophilic protease capable of degrading potentially contaminating nucleic acid and/or nucleic acid degrading enzymes in said working solution; and
maintaining said one or more working solution at a first temperature or temperature range at which said one or more mesophilic nuclease has enzymatic activity, but at which said at least one thermophilic protease has minimal or no proteolytic activity; and
storing said one or more working solution at a preferred temperature until required for use; and
completing purification prior to using said one or more working solution by raising the temperature of said one or more working solution to a second temperature or temperature range at which said at least one thermophilic protease has proteolytic activity; and
cooling said one or more working solution for use.

38. The method of claim 37 wherein said nucleic acid diagnostic techniques include polymerase chain reaction techniques.

39. The method of claim 37 or claim 38 wherein said one or more working solution may undergo more than one heat treatment step.

40. The method of any one of claims 8 to 39 wherein said one or more thermophilic protease exhibits autolytic activity or is denaturable at temperatures above about 90°C.

41. The method of claim 40 wherein said one or more thermophilic protease is EA1 protease and/or Ak 1 protease.

42. The method of claim 34 wherein said method comprises the additional step of maintaining said composition at a third temperature or temperature range sufficient to cause denaturation and/or autolysis of said at least one thermophilic protease.

43. The method of claim 37 wherein said method comprises the additional step of maintaining said one or more working solution at a third temperature or temperature range sufficient to cause denaturation and/or autolysis of said at least one thermophilic protease.

## Patentansprüche

1. Verwendung zumindest einer thermophilen Protease und eines oder mehrerer mesophiler Enzyme in Kombination bei der Entfernung zumindest einer Verunreinigung aus einer Zusammensetzung, wobei die zumindest eine thermophile Protease optimale proteolytische Aktivität bei oder über 65°C aufweist und das eine oder die mehreren mesophilen Enzyme optimale enzymatische Aktivität unter 65°C aufweisen, wobei die Verunreinigung ein Substrat eines oder mehrerer mesophiler Enzyme ist, wobei die zumindest eine thermophile Protease proteolytische Aktivität bezüglich des einen oder der mehreren mesophilen Enzyme aufweist, und wobei die Zusammensetzung keine Nucleinsäureprobe ist, die eine Nucleinsäure zum Testen umfasst.

2. Verwendung nach Anspruch 1, wobei die zumindest eine thermophile Protease aus der aus EA1, Ak1, NprS, NprT, BT1, YP-T, Aqualysin 1, NprM, Caldolysin, Caldolase, Rt41A, RT4A2 und Varianten davon mit optimaler proteolytischer Aktivität bei oder über 65 °C bestehenden Gruppe ausgewählt ist.

3. Verwendung nach Anspruch 2, wobei die thermophile Protease EA1 und/oder Ak1 oder eine Variante davon ist.

4. Verwendung nach einem der Ansprüche 1 bis 3, wobei das eine oder die mehreren mesophilen Enzyme Nucleinsäure-modifizierende Enzyme sind.

5. Verwendung nach Anspruch 4, wobei das Nucleinsäure-modifizierende Enzym aus der Nucleasen, Phosphatasen, Kinasen, Polymerasen, Synthetasen, Ligasen, Methylasen, Transferasen und Rekombinasen umfassenden Gruppe ausgewählt ist.

6. Verwendung nach einem der Ansprüche 1 bis 3, wobei das eine oder die mehreren mesophilen Enzyme aus der aus ATPasen, Cytochromen, Desaminasen, Dehydrogenasen, Decarboxylasen, Desaturasen, Dioxygenasen, Elastasen, Endopeptidasen, Flavoproteinen, Flippasen, Hydrogenasen, Hydrolasen, Hydroxylasen, Integrasen, Isomerasen, Kinasen, Ligasen, Lipasen, Lipoxygenasen, Lyasen, Mutasen, Oxidasen, Oxidoreductasen, Peptidasen und proteolytischen Enzymen, Permeasen, Peroxidasen, Phosphorylasen, Rekombinasen, Reductasen, Regulationsenzymen, Ribonucleasen, Sulfatasen, Synthasen, Synthetasen, Telomerasen und Transferasen bestehenden Gruppe ausgewählt sind.

7. Verwendung nach einem der Ansprüche 1 bis 6, wobei die Zusammensetzung in einer Polymerasekettenreaktion eingesetzt werden soll.

8. Verfahren zur Entfernung zumindest einer Verunreinigung aus einer Zusammensetzung, wobei die Verunreinigung ein Substrat eines oder mehrerer mesophiler Enzyme ist, wobei das Verfahren folgende Schritte umfasst:
Zusetzen des einen oder der mehreren mesophilen Enzyme und zumindest einer thermophilen Protease zur Zusammensetzung; und
Halten der Zusammensetzung auf einer ersten Temperatur oder in einem ersten Temperaturbereich, bei der/in dem das eine oder die mehreren mesophilen Enzyme enzymatische Aktivität aufweisen, bei denen die zumindest eine thermophile Protease aber minimale oder keine proteolytische Aktivität aufweist, und
Halten der Zusammensetzung auf einer zweiten Temperatur oder in einem zweiten Temperaturbereich, bei der/in dem die zumindest eine thermophile Protease proteolytische Aktivität aufweist; und
Halten der Zusammensetzung bei einer für eine Anwendung geeigneten Temperatur;
wobei das eine oder die mehreren mesophilen Enzyme keine Aktivität aufweisen, die zur Entfernung der Zellwände wirksam ist.

9. Verfahren nach Anspruch 8, wobei das Verfahren nach dem Halten auf einer zweiten Temperatur oder in einem zweiten Temperaturbereich eine oder mehrere Wiederholungen der folgenden zusätzlichen Schritte umfasst:
Zusetzen eines oder mehrerer mesophiler Enzyme zur Zusammensetzung; und
Halten der Zusammensetzung auf einer Temperatur oder in einem Temperaturbereich, bei der/in dem das eine oder die mehreren mesophilen Enzyme enzymatische Aktivität aufweisen, bei denen aber die zumindest eine thermophile Protease minimale oder keine proteolytische Aktivität aufweist; und
Halten der Zusammensetzung auf einer Temperatur oder in einem Temperaturbereich, bei der/in dem die zumindest eine thermophile Protease proteolytische Aktivität aufweist.

10. Verfahren nach Anspruch 8 oder Anspruch 9, wobei das Verfahren den zusätzlichen Schritt des Haltens der Zusammensetzung auf einer dritten Temperatur oder in einem dritten Temperaturbereich umfasst, die/der ausreicht, um eine Denaturierung und/oder Autolyse der zumindest einen thermophilen Protease auszulösen.

11. Verfahren nach einem der Ansprüche 8 bis 10, wobei die erste Temperatur oder der erste Temperaturbereich zwischen etwa 10 °C und etwa 65 °C liegt.

12. Verfahren nach einem der Ansprüche 8 bis 11, wobei die erste Temperatur oder der erste Temperaturbereich zwischen etwa 20 °C und etwa 50 °C liegt.

13. Verfahren nach einem der Ansprüche 8 bis 12, wobei die erste Temperatur oder der erste Temperaturbereich zwischen etwa 30 °C und etwa 40 °C liegt.

14. Verfahren nach einem der Ansprüche 8 bis 12, wobei die erste Temperatur oder der erste Temperaturbereich zwischen etwa 40 °C und etwa 65 °C liegt.

15. Verfahren nach einem der Ansprüche 8-10, 12 und 13, wobei die zweite Temperatur oder der zweite Temperaturbereich zwischen etwa 65 °C und etwa 85 °C liegt.

16. Verfahren nach Anspruch 15, wobei die zweite Temperatur oder der zweite Temperaturbereich zwischen etwa 65 °C und etwa 80 °C liegt.

17. Verfahren nach einem der Ansprüche 8 bis 16, wobei die zweite Temperatur oder der zweite Temperaturbereich zwischen etwa 70 °C und etwa 80 °C liegt.

18. Verfahren nach einem der Ansprüche 8 bis 17, wobei die zweite Temperatur etwa 75 °C beträgt.

19. Verfahren nach Anspruch 10, wobei die dritte Temperatur oder der dritte Temperaturbereich zwischen etwa 90 °C und etwa 105 °C liegt.

20. Verfahren nach Anspruch 19, wobei die dritte Temperatur oder der dritte Temperaturbereich zwischen etwa 90 °C und etwa 100 °C liegt.

21. Verfahren nach Anspruch 19, wobei die dritte Temperatur oder der dritte Temperaturbereich zwischen etwa 90 °C und etwa 95 °C liegt.

22. Verfahren nach Anspruch 21, wobei die dritte Temperatur etwa 95 °C beträgt.

23. Verfahren nach einem der Ansprüche 8 bis 22, wobei das eine oder die mehreren mesophilen Enzyme Nucleinsäure-modifizierende Enzyme sind.

24. Verfahren nach Anspruch 23, wobei das Enzym aus der aus Nucleasen, Phosphatasen, Kinasen, Polymerasen, Synthetasen, Ligasen, Methylasen, Transferasen und Rekombinasen bestehenden Gruppe ausgewählt ist.

25. Verfahren nach einem der Ansprüche 8 bis 22, wobei das eine oder die mehreren mesophilen Enzyme aus der aus ATPasen, Cytochromen, Desaminasen, Dehydrogenasen, Decarboxylasen, Desaturasen, Dioxygenasen, Elastasen, Endopeptidasen, Flavoproteinen, Flippasen, Hydrogenasen, Hydrolasen, Hydroxylasen, Integrasen, Isomerasen, Kinasen, Ligasen, Lipasen, Lipoxygenasen, Lyasen, Mutasen, Oxidasen, Oxidoreductasen, Peptidasen und proteolytischen Enzymen, Permeasen, Peroxidasen, Phosphorylasen, Rekombinasen, Reductasen, Regulationsenzymen, Ribonucleasen, Sulfatasen, Synthasen, Synthetasen, Telomerasen und Transferasen bestehenden Gruppe ausgewählt sind.

26. Verfahren nach einem der Ansprüche 8 bis 25, wobei die Zusammensetzung ein verschließbarer Behälter ist oder sich darin befindet.

27. Verfahren nach Anspruch 26, wobei der Behälter nach dem Zusatz des einen oder der mehreren mesophilen Enzyme und der zumindest einen thermophilen Protease und vor der Verwendung verschlossen bleibt.

28. Verfahren nach einem der Ansprüche 8 bis 27, wobei das Verfahren in einem geschlossenen Röhrensystem durchgeführt wird.

29. Verfahren nach einem der Ansprüche 8 bis 28, wobei das Verfahren automatisiert ist.

30. Verfahren nach Anspruch 29, wobei beim Verfahren ein Thermocycler eingesetzt wird.

31. Verfahren nach Anspruch 30, wobei beim Verfahren ein Thermocycler in Kombination mit robotergesteuerter Flüssigkeitshandhabung eingesetzt wird.

32. Verfahren nach einem der Ansprüche 8 bis 31, wobei die zumindest eine thermophile Protease aus der aus EA1, Ak1, NprS, NprT, BT1, YP-T, Aqualysin I, NprM, Caldolysin, Caldolase, Rt41A, RT4A2 und Varianten davon bestehenden Gruppe ausgewählt ist.

33. Verfahren nach Anspruch 32, wobei die thermophile Protease EA1 und/oder Ak1 oder eine Variante davon ist.

34. Verfahren zum Entfernen von Nucleinsäure aus einer Zusammensetzung, wobei das Verfahren folgende Schritte umfasst:
Zusetzen eines oder mehrerer mesophiler Nucleinsäure-abbauender Enzyme und zumindest einer thermophilen Protease zur Zusammensetzung; und
Halten der Zusammensetzung auf einer ersten Temperatur oder in einem ersten Temperaturbereich, bei der/in dem das eine oder die mehreren mesophilen Nucleinsäure-abbauenden Enzyme enzymatische Aktivität aufweisen, bei denen die zumindest eine thermophile Protease aber minimale oder keine proteolytische Aktivität aufweist, und
Halten der Zusammensetzung auf einer zweiten Temperatur oder in einem zweiten Temperaturbereich, bei der/in dem die zumindest eine thermophile Protease proteolytische Aktivität aufweist; und
Halten der Zusammensetzung bei einer für eine Anwendung geeigneten Temperatur.

35. Verfahren nach Anspruch 34, wobei das eine oder die mehreren mesophilen Nucleinsäure-abbauenden Enzyme Nucleasen sind.

36. Verfahren nach einem der Ansprüche 34 oder 35, wobei die Zusammensetzung zur Verwendung in einem PCR-Verfahren dient.

37. Verfahren zur Reinigung einer oder mehrerer Arbeitslösungen für Nucleinsäure-Diagnoseverfahren in einem geschlossenen Röhrensystem, wobei das Verfahren folgende Schritte umfasst:
Zusetzen einer oder mehrerer mesophiler Nucleasen und zumindest einer thermophilen Protease, die zum Abbau von möglicherweise verunreinigender Nucleinsäure und/oder Nucleinsäure-abbauenden Enzymen in der Arbeitslösung in der Lage ist, zu der einen oder den mehreren Arbeitslösungen; und
Halten der einen oder mehreren Arbeitslösungen auf einer ersten Temperatur oder in einem ersten Temperaturbereich, bei der/in dem die eine oder mehreren mesophilen Nucleasen enzymatische Aktivität aufweisen, bei denen die zumindest eine thermophile Protease aber minimale oder keine proteolytische Aktivität aufweist, und
Lagern der einen oder mehreren Arbeitslösungen bei einer bevorzugten Temperatur bis zur Verwendung; und
Vervollständigen der Reinigung vor der Verwendung der einen oder mehreren Arbeitslösungen durch Erhöhen der Temperatur der einen oder mehreren Arbeitslösungen auf eine zweite Temperatur oder einen zweiten Temperaturbereich, bei der/dem die zumindest eine thermophile Protease proteolytische Aktivität aufweist; und
Abkühlen der einen oder mehreren Arbeitslösungen für die Verwendung.

38. Verfahren nach Anspruch 37, wobei die Nucleinsäure-Diagnoseverfahren Polymerasekettenreaktionsverfahren umfassen.

39. Verfahren nach Anspruch 37 oder 38, wobei die eine oder mehreren Arbeitslösungen gegebenenfalls mehr als einen Wärmebehandlungsschritt durchlaufen.

40. Verfahren nach einem der Ansprüche 8 bis 39, wobei die eine oder mehreren thermophilen Proteasen autolytische Aktivität aufweisen oder bei Temperaturen über etwa 90 °C denaturierbar sind.

41. Verfahren nach Anspruch 40, wobei die oder mehreren thermophilen Proteasen EA1-Protease und/oder Ak1-Protease sind.

42. Verfahren nach Anspruch 34, wobei das Verfahren den zusätzlichen Schritt des Haltens der Zusammensetzung auf einer dritten Temperatur oder in einem dritten Temperaturbereich umfasst, die ausreichen, um eine Denaturierung und/oder Autolyse der zumindest einen thermophilen Protease auszulösen.

43. Verfahren nach Anspruch 37, wobei das Verfahren den zusätzlichen Schritt des Haltens der einen oder mehreren Arbeitslösungen auf einer dritten Temperatur oder in einem dritten Temperaturbereich umfasst, die ausreichen, um eine Denaturierung und/oder Autolyse der zumindest einen thermophilen Protease auszulösen.

## Revendications

1. Utilisation d'au moins une protéase thermophile et d'une ou de plusieurs enzymes mésophiles de manière combinée dans l'élimination d'au moins un contaminant d'une composition, dans laquelle la au moins une protéase thermophile présente une activité protéolytique optimale à une température supérieure ou égale à 65 °C et l'enzyme (les enzymes) mésophile(s) présente(nt) une activité enzymatique optimale en dessous de 65 °C, dans laquelle ledit contaminant est un substrat d'une ou de plusieurs enzymes mésophiles, dans laquelle la au moins une protéase thermophile présente une activité protéolytique envers l'enzyme (les enzymes) mésophile(s), et dans laquelle ladite composition n'est pas un échantillon d'acide nucléique comprenant un acide nucléique à tester.

2. Utilisation selon la revendication 1, dans laquelle ladite au moins une protéase thermophile est choisie dans le groupe constitué de l'EAl, de l'Ak1, de la NprS, de la NprT, de la BT1, de la YP-T, de l'aqualysine I, de la NprM, de la Caldolysine, de la Caldolase, de la Rt41A, de la RT4A2 et de leurs variantes présentant une activité protéolytique optimale à une température supérieure ou égale à 65 °C.

3. Utilisation selon la revendication 2, dans laquelle ladite protéase thermophile est de l'EA1 et/ou de l'Ak1 ou des variantes de celles-ci.

4. Utilisation selon l'une quelconque des revendications 1 à 3, dans laquelle lesdites une ou plusieurs enzymes mésophiles sont une enzyme de modification d'acide nucléique.

5. Utilisation selon la revendication 4, dans laquelle ladite enzyme de modification d'acide nucléique est choisie dans le groupe comprenant les nucléases, les phosphatases, les kinases, les polymérases, les synthétases, les ligases, les méthylases, les transférases et les recombinases.

6. Utilisation selon l'une quelconque des revendications 1 à 3, dans laquelle lesdites une ou plusieurs enzymes mésophiles sont choisies dans le groupe constitué des ATPases, des cytochromes, des désaminases, des déshydrogénases, des décarboxylases, des désaturases, des dioxygénases, des élastases, des endopeptidases, des flavoprotéines, des flippases, des hydrogénases, des hydrolases, des hydroxylases, des intégrases, des isomérases, des kinases, des ligases, des lipases, des lipoxygénases, des lyases, des mutases, des oxydases, des oxydoréductases, des peptidases et des enzymes protéolytiques, des perméases, des peroxydases, des phosphorylases, des recombinases, des réductases, des enzymes régulatrices, des ribonucléases, des sulfatases, des synthases, des synthétases, des télomérases et des transférases.

7. Utilisation selon l'une quelconque des revendications 1 à 6, dans laquelle ladite composition est à utiliser dans une réaction en chaîne par polymérase.

8. Procédé d'élimination d'au moins un contaminant d'une composition, dans lequel ledit contaminant est un substrat d'une ou de plusieurs enzymes mésophiles, ledit procédé comprenant les étapes consistant à:
ajouter à ladite composition lesdites une ou plusieurs enzymes mésophiles et au moins une protéase thermophile; et
maintenir ladite composition à une première température ou dans un premier intervalle de température où lesdites une ou plusieurs enzymes mésophiles présentent une activité enzymatique, mais où ladite au moins une protéase thermophile présente une activité protéolytique minime ou nulle; et
maintenir ladite composition à une seconde température ou dans un second intervalle de température où ladite au moins une protéase thermophile présente une activité protéolytique; et
maintenir ladite composition à une température adéquate pour l'utilisation;
dans lequel lesdites une ou plusieurs enzymes mésophiles ne présentent pas une activité suffisante pour enlever les parois cellulaires.

9. Procédé selon la revendication 8, ledit procédé comprenant, après ledit maintien à une seconde température ou dans un second intervalle de température, une ou plusieurs répétitions des étapes supplémentaires consistant à:
ajouter à ladite composition une ou plusieurs enzymes mésophiles; et
maintenir ladite composition à une température ou dans un intervalle de température où lesdites une ou plusieurs enzymes mésophiles présentent une activité enzymatique, mais où ladite au moins une protéase thermophile présente une activité protéolytique minime ou nulle; et
maintenir ladite composition à une température ou dans un intervalle de température où ladite au moins une protéase thermophile présente une activité protéolytique.

10. Procédé selon la revendication 8 ou la revendication 9, ledit procédé comprenant l'étape supplémentaire consistant à maintenir ladite composition à une troisième température ou dans un troisième intervalle de température suffisants pour entraîner la dénaturation et/ou l'autolyse de ladite au moins une protéase thermophile.

11. Procédé selon l'une quelconque des revendications 8 à 10, dans lequel ladite première température ou ledit premier intervalle de température sont compris entre environ 10 °C et environ 65 °C.

12. Procédé selon l'une quelconque des revendications 8 à 11, dans lequel ladite première température ou ledit premier intervalle de température sont compris entre environ 20 °C et environ 50 °C.

13. Procédé selon l'une quelconque des revendications 8 à 12, dans lequel ladite première température ou ledit premier intervalle de température sont compris entre environ 30 °C et environ 40 °C.

14. Procédé selon l'une quelconque des revendications 8 à 12, dans lequel ladite première température ou ledit premier intervalle de température sont compris entre environ 40 °C et environ 65 °C.

15. Procédé selon l'une quelconque des revendications 8 à 10, 12 et 13, dans lequel ladite seconde température ou ledit second intervalle de température sont compris entre environ 65 °C et environ 85 °C.

16. Procédé selon la revendication 15, dans lequel ladite seconde température ou ledit second intervalle de température sont compris entre environ 65 °C et environ 80 °C.

17. Procédé selon l'une quelconque des revendications 8 à 16, dans lequel ladite seconde température ou ledit second intervalle de température sont compris entre environ 70 °C et environ 80 °C.

18. Procédé selon l'une quelconque des revendications 8 à 17, dans lequel ladite seconde température est d'environ 75°C.

19. Procédé selon la revendication 10, dans lequel ladite troisième température ou ledit troisième intervalle de température sont compris entre environ 90°C et environ 105 °C.

20. Procédé selon la revendication 19, dans lequel ladite troisième température ou ledit troisième intervalle de température sont compris entre environ 90 °C et environ 100 °C**.**

21. Procédé selon la revendication 19, dans lequel ladite troisième température ou ledit troisième intervalle de température sont compris entre environ 90 °C et environ 95 °C.

22. Procédé selon la revendication 21, dans lequel ladite troisième température est d'environ 95 °C.

23. Procédé selon l'une quelconque des revendications 8 à 22, dans lequel lesdites une ou plusieurs enzymes mésophiles sont une enzyme de modification d'acide nucléique.

24. Procédé selon la revendication 23, dans lequel ladite enzyme est choisie dans le groupe constitué des nucléases, des phosphatases, des kinases, des polymérases, des synthétases, des ligases, des méthylases, des transférases et des recombinases.

25. Procédé selon l'une quelconque des revendications 8 à 22, dans lequel lesdites une ou plusieurs enzymes mésophiles sont choisies dans le groupe constitué des ATPases, des cytochromes, des désaminases, des déshydrogénases, des décarboxylases, des désaturases, des dioxygénases, des élastases, des endopeptidases, des flavoprotéines, des flippases, des hydrogénases, des hydrolases, des hydroxylases, des intégrases, des isomérases, des kinases, des ligases, des lipases, des lipoxygénases, des lyases, des mutases, des oxydases, des oxydoréductases, des peptidases et des enzymes protéolytiques, des perméases, des peroxydases, des phosphorylases, des recombinases, des réductases, des enzymes régulatrices, des ribonucléases, des sulfatases, des synthases, des synthétases, des télomérases et des transférases.

26. Procédé selon l'une quelconque des revendications 8 à 25, dans lequel ladite composition est un récipient pouvant se fermer ou est située dans un récipient pouvant se fermer.

27. Procédé selon la revendication 26, dans lequel ledit récipient reste fermé après addition desdites une ou plusieurs enzymes mésophiles et de ladite au moins une protéase thermophile et avant utilisation.

28. Procédé selon l'une quelconque des revendications 8 à 27, ledit procédé étant réalisé dans un système de tube fermé.

29. Procédé selon l'une quelconque des revendications 8 à 28, ledit procédé étant automatique.

30. Procédé selon la revendication 29, ledit procédé utilisant un thermocycleur.

31. Procédé selon la revendication 30, ledit procédé utilisant un thermocycleur en combinaison avec un système robotisé de manipulation des liquides.

32. Procédé selon l'une quelconque des revendications 8 à 31, dans lequel ladite au moins une protéase thermophile est choisie dans le groupe constitué de l'EA1, de l'Ak1, de la NprS, de la NprT, de la BT1, de la YP-T, de l'aqualysine I, de la NprM, de la Caldolysine, de la Caldolase, de la Rt41A, de la RT4A2 et de leurs variantes.

33. Procédé selon la revendication 32, dans lequel ladite protéase thermophile est de l'EA1 et/ou de l'Ak1 ou des variantes de celles-ci.

34. Procédé d'élimination d'un acide nucléique d'une composition, ledit procédé comprenant les étapes consistant à:
ajouter à ladite composition une ou plusieurs enzymes de dégradation d'acide nucléique mésophiles et au moins une protéase thermophile; et
maintenir ladite composition à une première température ou dans un premier intervalle de température où lesdites une ou plusieurs enzymes de dégradation d'acide nucléique mésophiles présentent une activité enzymatique, mais où ladite au moins une protéase thermophile présente une activité protéolytique minime ou nulle; et
maintenir ladite composition à une seconde température ou dans un second intervalle de température où ladite au moins une protéase thermophile présente une activité protéolytique; et
maintenir ladite composition à une température adéquate pour l'utilisation.

35. Procédé selon la revendication 34, dans lequel lesdites une ou plusieurs enzymes de dégradation d'acide nucléique mésophiles sont une nucléase.

36. Procédé selon l'une quelconque des revendications 34 ou 35, dans lequel ladite composition est à utiliser dans une procédure de réaction en chaîne par polymérase.

37. Procédé de purification d'une ou de plusieurs solutions de travail pour techniques de diagnostic d'acide nucléique dans un système de tube fermé, ledit procédé comprenant les étapes consistant à:
ajouter auxdites une ou plusieurs solutions de travail une ou plusieurs nucléases mésophiles et au moins une protéase thermophile capable de dégrader un acide nucléique potentiellement contaminant et/ou des enzymes de dégradation d'acide nucléique dans ladite solution de travail; et
maintenir lesdites une ou plusieurs solutions de travail à une première température ou dans un premier intervalle de température où lesdites une ou plusieurs nucléases mésophiles présentent une activité enzymatique, mais où ladite au moins une protéase thermophile présente une activité protéolytique minime ou nulle; et
stocker lesdites une ou plusieurs solutions de travail à une température préférée jusqu'à utilisation; et
réaliser une purification avant d'utiliser lesdites une ou plusieurs solutions de travail en augmentant la température desdites une ou plusieurs solutions de travail à une seconde température ou dans un second intervalle de température où ladite au moins une protéase thermophile présente une activité protéolytique; et
refroidir lesdites une ou plusieurs solutions de travail pour l'utilisation.

38. Procédé selon la revendication 37, dans lequel lesdites techniques de diagnostic d'acide nucléique comprennent des techniques de réaction en chaîne par polymérase.

39. Procédé selon la revendication 37 ou la revendication 38, dans lequel lesdites une ou plusieurs solutions de travail peuvent subir plus d'une étape de traitement thermique.

40. Procédé selon l'une quelconque des revendications 8 à 39, dans lequel lesdites une ou plusieurs protéases thermophiles présentent une activité autolytique ou sont dénaturables à des températures supérieures à environ 90 °C.

41. Procédé selon la revendication 40, dans lequel lesdites une ou plusieurs protéases thermophiles sont une protéase d'EA1 et/ou une protéase d'Ak1.

42. Procédé selon la revendication 34, ledit procédé comprenant l'étape supplémentaire consistant à maintenir ladite composition à une troisième température ou dans un troisième intervalle de température suffisants pour entraîner la dénaturation et/ou l'autolyse de ladite au moins une protéase thermophile.

43. Procédé selon la revendication 37, ledit procédé comprenant l'étape supplémentaire consistant à maintenir lesdites une ou plusieurs solutions de travail à une troisième température ou dans un troisième intervalle de température suffisants pour entraîner la dénaturation et/ou l'autolyse de ladite au moins une protéase thermophile.
